(19) Europäisches Patentamt
European Patent Office
Office européen des brevets

(11) Veröffentlichungsnummer: **0 020 437**
**B1**

(12) # EUROPÄISCHE PATENTSCHRIFT

(45) Veröffentlichungstag der Patentschrift: **23.02.83**

(51) Int. Cl.³: **C 07 D 231/12,** A 01 N 43/56

(21) Anmeldenummer: **79901217.4**

(22) Anmeldetag: **04.10.79**

(86) Internationale Anmeldenummer: **PCT/EP 79/00076**

(87) Internationale Veröffentlichungsnummer: **WO 80/00701 (17.04.80** Gazette **80/8)**

(54) **Halogenacetanilide, ihre Herstellung und diese enthaltende Herbizide.**

(30) Priorität: **07.10.78 DE 2843869**

(43) Veröffentlichungstag der Anmeldung: **07.01.81 Patentblatt 81/1**

(45) Bekanntmachung des Hinweises auf die Patenterteilung: **23.02.83 Patentblatt 83/8**

(84) Benannte Vertragsstaaten: **FR**

(56) Entgegenhaltungen: **keine**

(73) Patentinhaber: **BASF Aktiengesellschaft, Carl-Bosch-Strasse 38, D-6700 Ludwigshafen (DE)**

(72) Erfinder: **EICKEN, Karl, Dr., Waldstrasse 63, D-6706 Wachenheim (DE)**
Erfinder: **ROHR, Wolfgang, Dr., Gontardstr. 4, D-6800 Mannheim (DE)**
Erfinder: **GOETZ, Norbert, Dr., Schoefferstr. 25, D-6520 Worms (DE)**
Erfinder: **WUERZER, Bruno, Dr., Wilhelm Busch-Str. 55, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Halogenacetanilide, ihre Herstellung und diese enthaltende Herbizide

Anwendungsgebiet der Erfindung

Die Erfindung wird bei der Bekämpfung von unerwünschten Pflanzen angewendet.

Charakteristik der bekannten technischen Lösungen

Halogenacetanilide mit herbiziden oder wachstumsregulierenden Eigenschaften sind bekannt. Eine beachtliche wirtschaftliche Bedeutung erlangte das 2-Chlor-2′,6′-diethyl-N-methoxymethyl-acetanilid (US-PS 3 547 620). Als weiterer Wirkstoff ist das 2-Chlor-2′-äthyl-6′-methyl-N-(1′-methoxyprop-2′-yl)-acetanilid bekannt (DE-AS 2 328 340). Dieses besitzt neben einer guten Wirkung gegen unerwünschte Gräser eine gute Dauerwirkung im Boden.

Ziel der Erfindung

Ziel der Erfindung ist die Entwicklung herbizider Mittel mit gesteigerter Wirksamkeit.

Darlegung des Wesens der Erfindung

Der Erfindung liegt die Aufgabe zugrunde, ein neues herbizides Mittel bereitzustellen.

Es wurde gefunden, dass ein Herbizid, das ein Halogenacetanilid der Formel I

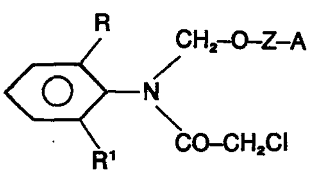

(I)

in der

R und $R^1$ gleich oder verschieden sind und Alkyl mit 1 bis 4 Kohlenstoffatomen,

Z eine Methylengruppe oder eine gegebenenfalls durch ein bis zwei Methylgruppen substituierte Äthylengruppe und

A eine über ein Ringstickstoffatom gebundenes Pyrazolyl, enthält, eine verbreiterte herbizide Wirkung bei guter Selektivität bei wichtigen Kulturpflanzen aufweist.

Die neuen Wirkstoffe sind je nach Zielsetzung und Dosis geeignet zur selektiven Bekämpfung von unerwünschten Pflanzen in bestimmten Kulturpflanzenbeständen, bestehend aus krautigen oder verholzenden Pflanzenarten, zur Wachstumsregulierung durch Hemmung des Pflanzenwachstums oder bei entsprechenden hohen Aufwandmengen zur totalen Bekämpfung des Pflanzenwuchses.

Für die Symbole R, $R^1$, Z und A kommen in Betracht:

Für R bzw. $R^1$ gleiche oder verschiedene Alkylreste mit 1 bis 4 Kohlenstoffatomen wie Methyl, Äthyl, n-Propyl, i-Propyl, n-Butyl, sec.-Butyl, tert.-Butyl;

für Z eine Methylengruppe oder eine gegebenenfalls durch ein oder zwei Methylgruppen substituierte Äthylenkette wie Äthylen ($-CH_2-CH_2-$), 1-Methyläthylen, 2-Methyläthylen, 1,2-Dimethyläthylen;

für A ein über ein Ringstickstoffatom gebundenes Pyrazolyl.

Die neuen Halogenacetanilide können nach folgendem Verfahren hergestellt werden:

Sie werden durch Umsetzung von 2-Chlor-N-chlor-methyl-acetaniliden der Formel II mit einem Alkohol der Formel III nach folgender Reaktionsgleichung erhalten.

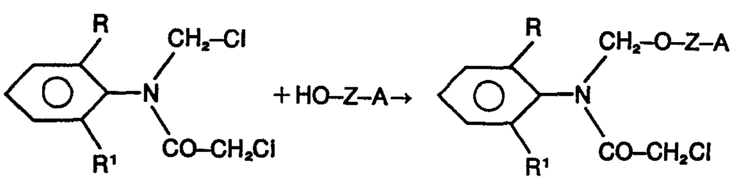


(II) (III) (I) +HCl

Dabei haben die Substituenten R, $R^1$, Z und A die oben angegebene Bedeutung. Einige der 2-Chlor-N-chlormethyl-acetanilide der Formel II sind aus der US-PS 3 637 847 bekannt; andere können auf analoge Weise durch Umsetzung der entsprechenden Azomethine mit Chloracetylchlorid hergestellt werden.

Der Alkohol der Formel III wird zweckmässig in mindestens molarer Menge, bezogen auf das 2-Chlor-N-chlor-methylacetanilid, eingesetzt.

Der bei der Umsetzung freiwerdende Chlorwasserstoff kann als Gas entfernt oder durch geeignete Bindemittel, wie organische Basen z.B. tertiäre Amine oder anorganische Basen, beispielsweise Alkalicarbonate abgefangen werden. Das Bindemittel für Chlorwasserstoff wird in mindestens molarer Menge, bezogen auf eingesetztes 2-Chlor-N-chlormethylacetanilid, verwendet.

Bei der Umsetzung von 2-Chlor-N-chlor-methylacetaniliden der Formel II mit solchen Alkoholen der Formel III, in denen Z eine Methylengruppe ($-CH_2-$) bedeutet, ist es besonders vorteilhaft, die Alkohole in Form ihrer Alkalisalze zu verwenden. Diese Alkalisalze lassen sich allgemein durch

Umsetzung der 1-Hydroxymethyl-pyrazole mit starken Basen wie metallorganische Verbindungen z.B. Butyllithium, Methylmagnesiumchlorid; wie Alkalimetallhydride, z.B. Natriumhydrid; wie Alkalimetallamide z.B. Natriumamid, oder wie Alkalimetallalkoholate z.B. Natriummethylat unter Abspaltung von Wasserstoff, Alkan, Ammoniak oder Methanol herstellen.

Es ist vorteilhaft, die Reaktion in einem Lösungsmittel durchzuführen, das gegenüber 2-Chlor-N-chlormethylacetaniliden inert ist. Hierzu eignen sich Kohlenwasserstoffe wie Toluol, Xylol; Äther wie Diäthyläther, tert.-Butyl-methyläther, 1,2-Dimethoxyäthan, Tetrahydrofuran, Dioxan; Ester wie Essigester, Butylacetat, Nitrile wie Acetonitril, Propionnitril, Sulfone wie Dimethylsulfoxid, Tetrahydrothiophen-1,1-dioxid, sekundäre Amide wie N,N-Dimethylformamid, N,N-Diäthyl-formamid. Auch Gemische dieser Lösungsmittel können verwendet werden. Insbesondere bei Umsetzungen von Alkalisalzen der 1-Hydroxymethyl-pyrazole der Formel III mit Z in der Bedeutung Methylen ($-CH_2-$) ist es vorteilhaft in aprotisch polaren Lösungsmitteln oder Gemischen mit diesen Lösungsmitteln zu arbeiten.

Die Umsetzungen der 2-Chlor-N-chlormehtylacetanilide der Formel II mit den Alkoholen der Formel III bzw. den Alkalimetallsalzen dieser Alkohole werden bei Temperaturen zwischen −30 bis +50 °C, vorzugsweise bei Raumtemperatur, durchgeführt. Reaktionsprodukte werden – gegebenenfalls nach Abtrennen der gebildeten Nebenprodukte wie Alkalimetallchlorid bzw. Trialkylammoniumchloriden und gegebenenfalls nach Austausch des polaren, aprotischen Solvens durch ein mit Wasser nicht mischbares Lösungsmittel – in üblicher Weise isoliert.

Ein Teil der für die Herstellung der erfindungsgemässen Halogenacetanilide der Formel I verwendeten Alkohole der Formel III ist bekannt wie z.B. 1-Hydroxy-methylpyrazole und 1-(2-Hydroxyäthyl)-pyrazole [Chem. Ber. 85, 820 (1952); J. Chem. Soc. 1960, 5272].

Eine einfache Herstellung von 1-(2-Hydroxyäthyl) bzw. 1-(2-Hydroxypropyl)-pyrazol ist die Umsetzung von Epoxiden mit Pyrazolen in an sich bekannter Weise nach folgender Reaktionsgleichung (vgl. Herstellbeispiel A):

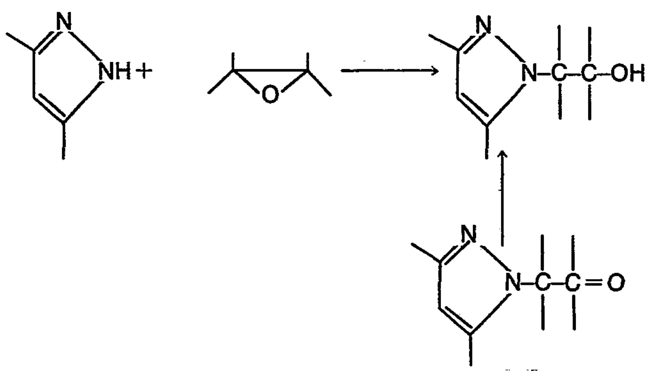

Ein weiteres Herstellverfahren dieser Alkohole besteht in der Reduktion entsprechender α-Pyrazol-1-yl-ketone bzw. -aldehyde z.B. mit komplexen Hydriden wie Natriumborhydrid in Alkohol oder Tetrahydrofuran.

In den Beispielen verhalten sich Gewichtsteile zu Volumenteile wie Kilogramm zu Liter.

Beispiel A

136 Teile (Gewichtsteile) Pyrazol in 500 Gewichtsteilen 4-Methylmorpholin gelöst, werden mit 2 Volumenteilen Wasser versetzt und in einem Rührautoklav mit einem Inhalt von 1000 Raumteilen auf 140 °C erhitzt. Nach Erreichen dieser Temperatur werden 100 Gewichtsteile Äthylenoxid aufgepresst. Man lässt anschliessend 5 Stunden lang bei 140 °C nachreagieren. Die erhaltene Mischung wird destillativ aufgearbeitet, wobei man 195 Gewichtsteile 1-(2-Hydroxyäthyl)-pyrazol, $Kp_{0,01}$ = 87 °C erhält; Ausbeute = 87% d.Th.

In analoger Weise kann auch das 1-(2-Hydroxypropyl)-pyrazol hergestellt werden:

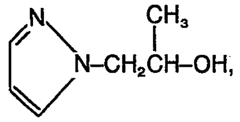

$Kp_{0,01} = 66$ °C

Die folgenden Beispiele erläutern die Herstellung der neuen Halogenacetanilide.

Beispiel 1

In eine Lösung von 14,7 Gewichtsteilen 1-Hydroxymethylpyrazol in 65 Volumenteilen trockenem Tetrahydrofuran werden bei 15–20 °C 3,6 Gewichtsteile Natriumhydrid portionsweise eingetragen und bis zur Beendigung der Wasserstoffentwicklung nachgerührt (2 Stunden). Zu dieser Mischung wird eine Lösung von 36,9 Gewichtsteilen 2-Chlor-2′,6′-dimethyl-N-chlormethylacetanilid in 100 Volumenteilen trockenem Acetonitril bei −10 °C zugetropft und über Nacht bei Raumtemperatur (20 °C) nachgerührt. Nach dem Verdampfen der Lösungsmittel im Vakuum wird der Rückstand in 100 Volumenteilen Methylenchlorid gelöst, nacheinander mit Wasser, 1n HCl-Lösung, gesättigter Bicarbonat-Lösung und zweimal mit Wasser gewaschen, über Natriumsulfat getrocknet und filtriert. Das Filtrat wird mit 4 Gewichtsteilen Kieselgel und 2 Gewichtsteilen Aktivkohle 2 Stunden gerührt und nach dem Absaugen bei 50 °C im Vakuum eingeengt. Der Rückstand ergibt nach dem Abkühlen 32,6 Gewichtsteile 2-Chlor-2′,6′-dimethyl-N-(pyrazol-1-yl-methylenoxymethyl)-acetanilid mit dem Fp. 73–74 °C (Wirkstoff 1).

$C_{15}H_{18}ClN_3O_7$ (MG 308)
Ber.: C 58,5 H 5,9 N 13,7
Gef.: C 57,7 H 5,8 N 13,7

Folgende Verbindungen werden analog hergestellt:

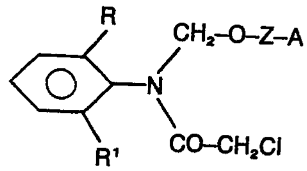

| Wirkstoff Nr. | R | $R^1$ | Z | A | $Fp/n_D$ |
|---|---|---|---|---|---|
| 2 | $CH_3$ | $C_2H_5$ | $CH_2$ | 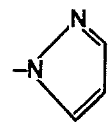 | Öl |
| 3 | $CH_3$ | $CH_3$ | $CH_2-CH_2$ | 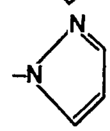 | 64 °C |
| 4 | $CH_3$ | $C_2H_5$ | $CH_2-CH_2$ | 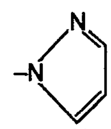 | Öl |
| 5 | $CH_3$ | $CH_3$ | 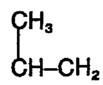 | 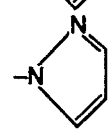 | Öl |
| 6 | $CH_3$ | $C_2H_5$ | 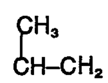 | 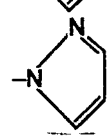 | 1,5481 |

Die erfindungsgemässen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken, sie sollen in jedem Fall eine feine und gleichmässige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Äthanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyäthylen-Fettalkohol-Äther, Alkylsulfonate und Arylsulfonate) und Dispergiermittel, wie Lignin-, Sulfitablaugen und Methylcellulose.

Die Mittel enthalten im allgemeinen zwischen 0,1 und 95 Gewichtsprozent Wirkstoff, vorzugsweise zwischen 0,5 und 90.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Die Aufwandmengen liegen nach Art des gewünschten Effektes zwischen 0,1 und 15 oder mehr, vorzugsweise jedoch zwischen 0,25 und 3 kg Wirkstoff pro Hektar.

Die neuen herbiziden Halogenacetanilide können mit zahlreichen Vertretern anderer herbizider oder wachstumsregulierender Wirkstoffgruppen gemischt und gemeinsam ausgebracht werden. Beispielsweise kommen als Mischungskomponente Diazine, Benzothiadiazinone, 2,6-Dinitroaniline, N-Phenyl-carbamate, Biscarbamate, Thiolcarbamate, Halogencarbonsäuren, Triazine, Amide, Harnstoffe, Diphenyläther, Triazinone, Uracile, Benzofuranderivate und andere in Betracht. Solche Kombinationen dienen zur Verbreiterung des Wirkungsspektrums und erzielen zuweilen synergistische Effekte.

Ausserdem ist es nützlich, die neuen erfindungsgemässen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralstofflösungen, welche zur Behebung von Ernährungs- oder Spurenelementmängeln eingesetzt werden.

Zur Aktivierung der herbiziden Wirkung können Netz- und Haftmittel sowie nichtphytotoxische Öle zugesetzt werden.

Der Einfluss verschiedener Vertreter der erfindungsgemässen Verbindungen auf das Wachstum von unerwünschten und erwünschten Pflanzen im Vergleich zu bekannten, chemisch ähnlichen Wirkstoffen wird in den folgenden Versuchen demonstriert. Die Versuchsserien wurden im Gewächshaus und im Freiland durchgeführt.

Ausführungsbeispiele

Gewächshausversuche

Als Kulturgefässe dienten Plastikblumentöpfe mit 300 $cm^3$ Inhalt und lehmiger Sand mit etwa 1,5% Humus als Substrat. Die Samen der Testpflanzen entsprechend Tabelle 1 wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Nach dem Aufbringen der Mittel wurden die Gefässe leicht beregnet, um Keimung und Wachstum in Gang zu bringen und die chemischen Mittel zu aktivieren. Danach deckte man die Gefässe mit durchsichtigen Plastikhauben ab, bis die Pflanzen angewachsen waren. Diese Abdekkung bewirkte ein gleichmässiges Keimen der Testpflanzen, sofern dies nicht durch die Chemikalien beeinträchtigt wurde.

Die Aufstellung der Gefässe erfolgte im Gewächshaus, wobei für wärmeliebende Arten wärmere Bereiche (25 bis 40 °C) und für solche gemässigter Klimata 15 bis 30 °C bevorzugt wurden. Die Versuchsperiode erstreckte sich über 4 bis 6 Wochen. Während dieser Zeit wurden die Pflanzen gepflegt und ihre Reaktion auf die einzelnen Behandlungen ausgewertet. Die Tabelle 2 enthält die Prüfsubstanzen, die jeweiligen Dosierungen in kg/ha Aktivsubstanz und die Testpflanzenarten. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Sprossteile.

Freilandversuche

Es handelt sich um Kleinparzellenversuche auf Standorten mit lehmigem Sand vom pH 6 und 1 bis 1,5% Humusgehalt. Es werden Vorauflaufanwendungen beschrieben, welche unmittelbar bis spätestens 3 Tage nach der Saat erfolgten. Die Kulturpflanzen wurden in Reihen gesät. Die Unkrautflora war natürlich vorkommend. Die Substanzen wurden in Wasser als Träger- und Verteilermedium emulgiert oder suspendiert und mit Hilfe einer motorgetriebenen, auf einen Geräteträger montierten Parzellenspritze ausgebracht. Bei Fehlen natürlicher Niederschläge wurde beregnet, um Keimung und Wachstum von Nutzpflanzen und Unkräutern zu gewährleisten. Alle Versuche liefen über mehrere Wochen. In diesem Zeitraum wurde die Bewertung ebenfalls unter Benutzung der Skala von 0 bis 100 vorgenommen.

Ergebnis

Die selektive herbizide Wirkung der erfindungsgemässen Verbindungen bei Vor- und Nachauflaufanwendungen geht aus den beigefügten Tabellen hervor.

Tabelle 1 – Liste der Testpflanzen

| Botanischer Name | Abkürzung in Tabellen | Deutscher Name | Englische Bezeichnung |
|---|---|---|---|
| Alopecurus myosuroides | Alopec. myos. | Ackerfuchsschwanz | slender foxtail |
| Amaranthus retroflexus | Amar. retr. | zurückgekrümmter Fuchsschwanz | pigweed |
| Avena fatua | – | Flughafer | wild oats |
| Beta vulgaris | Beta vulg. | Zuckerrübe | sugerbeet |
| Brassica napus | – | Raps | rape seed |
| Chenopodium album | Chenop. album | Weisser Gänsefuss | lamsquarters |
| Cyperus esculentus | Cyperus esc. | Erdmandel | yellow nutsedge |
| Echinochloa crus galli | Echin. c. g. | Hühnerhirse | barnyardgrass |
| Galinsoga spp. | Galin. spp. | Franzosenkrautarten | – |
| Gossypium hirsutum | Gossyp. hirs. | Baumwolle | cotton |
| Lamium spp. | – | Taubnesselarten | dead-nettle |
| Setaria spp. | – | Borstenhirseart | foxtail spp. |
| Solanum nigrum | Solan. nigrum | Schwarzer Nachtschatten | black nightshade |
| Stellaria media | Stell. med. | Vogelsternmiere | chickweed |
| Zea mays | – | Mais | Indian corn |

Tabelle 2 – Selektive herbizide Wirkung neuer Halogenacetanilide bei Vorauflaufanwendung im Gewächshaus

| Wirkstoff Nr. | kg/ha a.S. | Testpflanzen und Schädigung % Brassica napus | Alopec. myos. | Avena fatua | Amar. retr. | Echin. c.g. | Setaria spp. | Solan. nigrum |
|---|---|---|---|---|---|---|---|---|
| 4 | 2,0 | 10 | 80 | 80 | 95 | 80 | 95 | 90 |
| 5 | 2,0 | 0 | 100 | 95 | 90 | 95 | 98 | 100 |
| 6 | 2,0 | 20 | 100 | 95 | 100 | 98 | 80 | 100 |
| 1 | 0,25 | 0 | 98 | 82 | – | 98 | 100 | 100 |

Tabelle 3 – Bekämpfung von unerwünschten Pflanzen bei Nachauflaufanwendung im Gewächshaus

| Wirkstoff Nr. | kg/ha a.S. | Testpflanzen und Schädigung % Beta vulg. | Gossyp. hirs. | Alopec. myos. | Avena fatua | Cyperus esc. | Setaria spp. |
|---|---|---|---|---|---|---|---|
| 4 | 2,0 | o | 0 | 80 | 90 | 50 | 85 |
| 5 | 2,0 | 0 | 0 | 80 | 80 | 45 | 85 |
| 2 | 1,0 | 20 | 10 | 95 | 90 | 55 | 78 |

Tabelle 4 – Neue Halogenacetanilide bei selektiver Unkrautbekämpfung in Mais bei Vorauflaufanwendung im Freiland

| Wirkstoff Nr. | | kg/ha a.S. | Testpflanzen und Schädigung % Zea mays | Echin. c.g. | Chenop. album | Galin. spp. | Stell. med. | Lamium spp. |
|---|---|---|---|---|---|---|---|---|
| 2 | | 1,0 | 3 | 98 | 85 | 100 | 100 | 97 |
| | 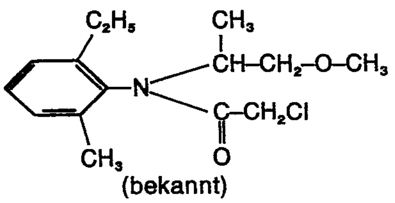 (bekannt) | 1,0 | 0 | 99 | 42 | 70 | 53 | 67 |
| | 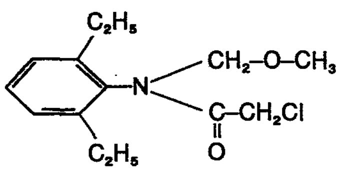 (bekannt) | 1,0 | 0 | 94 | 51 | 90 | 47 | 60 |

Beispiel 2

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel 3

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgiessen und feines Verteilen der Lösung in 100 000 Gewichtsteilen Wasser erhält man eine wässrige Dispersion, die 0,02 Gewichtsprozent des Wirkstoffs enthält.

Beispiel 4

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

Beispiel 5

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

Beispiel 6

20 Gewichtsteile des Wirkstoffs 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 7

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 8

30 Gewichtsteile der Verbindung 4 werden mit einer Mischung aus 92 Gewichtsteilen pulverförmigem Kiselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 9

40 Gewichtsteile des Wirkstoffs 1 werden mit 10 Teilen Natriumsalz eines Phenolsulfonsäure-harnstoff-formalkdehyd-Kondensats, 2 Teilen Kieselgel und 48 Teilen Wasser innig vermischt. Man erhält eine stabile wässrige Dispersion. Durch Verdünnen mit 100 000 Gewichtsteilen Wasser erhält man eine wässrige Dispersion, die 0,04 Gewichtsprozent Wirkstoff enthält.

Beispiel 10

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche**

1. Halogenacetanilid der Formel I

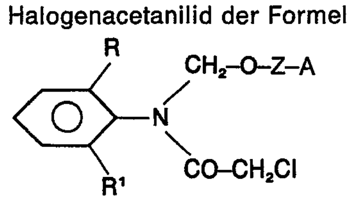

(I)

in der

R und $R^1$ gleich oder verschieden sind und Alkyl mit 1 bis 4 Kohlenstoffatomen,

Z eine Methylengruppe oder eine gegebenenfalls durch ein bis zwei Methylgruppen substituierte Äthylengruppe und

A ein über ein Ringstickstoffatom gebundenes Pyrazolyl bedeutet.

Halogenacetanilid gemäss Anspruch 1, nämlich 2-Chlor-2',6'-dimethyl-N-(pyrazol-1-yl-methylenoxymethyl)-acetanilid, 2-Chlor-2'-methyl-6'-äthyl-N-(pyrazol-1-yl-methylenoxy-methyl)-acetanilid.

3. Verfahren zur Herstellung eines Halogenacetanilids gemäss Anspruch 1, dadurch gekennzeichnet, dass man ein Halogenacetanilid der Formel II

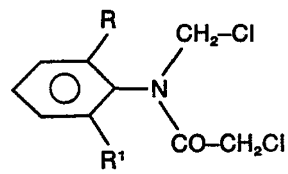

(II)

in der R und $R^1$ die im Anspruch 1 genannten Bedeutungen haben, umsetzt mit einem Alkohol der Formel III

$$HO\text{–}Z\text{–}A \qquad (III)$$

in der Z und A die im Anspruch 1 genannten Bedeutungen haben, wobei man den entstehenden Chlorwasserstoff gegebenenfalls an ein Bindemittel für Chlorwasserstoff bindet.

4. Herbizid, enthaltend ein Halogenacetanilid gemäss Anspruch 1.

5. Herbizid, enthaltend einen festen oder flüssigen Trägerstoff und ein Halogenacetanilid gemäss Anspruch 1.

6. Herbizid gemäss Anspruch 4, enthaltend ein Halogenacetanilid gemäss Anspruch 2, nämlich 2-Chlor-2',6'-dimethyl-N-(pyrazol-1-yl-methylenoxymethyl)-acetanilid, 2-Chlor-2'-mehtyl-6'-äthyl-N-(pyrazol-1-yl-methylenoxymethyl)-acetanilid.

7. Verfahren zur Herstellung eines Herbizids, dadurch gekennzeichnet, dass man einen festen oder flüssigen Trägerstoff vermischt mit einem Halogenacetanilid gemäss Anspruch 1.

8. Verfahren zur Bekämpfung unerwünschten Pflanzenwuchses, dadurch gekennzeichnet, dass man die Pflanzen oder den Boden behandelt mit einem Halogenacetanilid gemäss Anspruch 1.

**Claims**

1. A haloacetanilide of the formula I

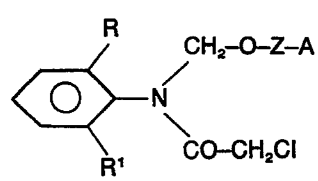

, (I)

where R and $R^1$ are identical or different and each denotes alkyl of 1 to 4 carbon atoms, Z denotes methylene, or ethylene which is unsubstituted or substituted by 1 or 2 methyl groups, and A denotes pyrazolyl which is linked via a ring nitrogen atom.

2. A haloacetanilide as claimed in claim 1, namely 2-chloro-2′-,6′-dimethyl-N-(pyrazol-1-yl-methylenoxymethyl)-acetanilide and 2-chloro-2′-methyl-6′-ethyl-N-(pyrazol-1-yl)-methylenoxymethyl)-acetanilide.

3. A process for preparing a haloacetanilide as claimed in claim 1, wherein a haloacetanilide of the formula II

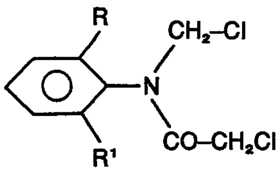 , (II)

where R an $R^1$ have the meanings given in claim 1, is reacted with an alcohol of the formula III

HO–Z–A (III)

where Z and A have the meanings given in claim 1, the hydrogen chloride which forms being, if desired, bound to a hydrogen chloride binder.

4. A herbicide containing a haloacetanilide as claimed in claim 1.

5. A herbicide containing a solid or liquid carrier and a haloacetanilide as claimed in claim 1.

6. A herbicide as claimed in claim 4 containing a haloacetanilide as claimed in claim 2, namely 2-chloro-2′,6′-dimethyl-N-(pyrazol-1-yl-methylenoxymethyl)-acetanilide and 2-chloro-2′-methyl-6′-ethyl-N-(pyrazol-1-yl-methylenoxymethyl)-acetanilide.

7. A process for the preparation of a herbicide wherein a solid or liquid carrier is mixed with a haloacetanilide as claimed in claim 1.

8. A process for combatting unwanted plant growth wherein the plants or the soil are treated with a haloacetanilide as claimed in claim 1.

**Revendications**

1. Halogénacétanilide de formule I

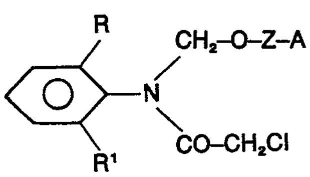 (I)

dans laquelle

R et $R^1$ sont identiques ou différents et représentent alkyle ayant 1 à 4 atomes de carbone;

Z un groupe méthyléne ou un groupe éthylène éventuellement substitué par un à deux groupes méthyle; et

A représente pyrazolyle lié par l'intermédiaire d'un atome d'azote de cycle.

2. Halogénacétanilide selon la revendication 1, à savoir 2-chloro-2′,6′-diméthyl-N-(pyrazol-1-yl-méthylènoxyméthyl)-acétanilide, 2-chloro-2′-méthyl-6′-éthyl-N-(pyrazol-1-yl-méthylènoxyméthyl)-acétanilide.

3. Procédé de préparation d'un halogénacétanilide selon la revendication 1, caractérisé par le fait qu'on fait réagir un halogénacétanilide de formule II

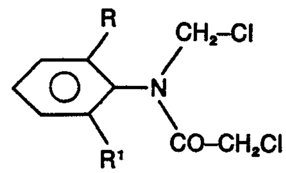 (II)

dans laquelle R et $R^1$ ont les significations données dans la revendication 1, avec un alcool de formule III

HO–Z–A (III)

dans laquelle Z et A ont les significations données dans la revendication 1, l'acide chlorhydrique produit étant éventuellement lié à un liant pour acide chlorhydrique.

4. Herbicide, contenant un halogénacétanilide selon la revendication 1.

5. Herbicide, contenant un support solide ou liquide et un halogénacétanilide selon la revendication 1.

6. Herbicide selon la revendication 4, contenant un halogénacétanilide selon la revendication 2 à savoir: 2-chloro-2′,6′-diméthyl-N-(pyrazol-1-yl-méthylènoxyméthyl)-acétanilide, 2-chloro-2′-méthyl-6′-éthyl-N-(pyrazol-1-yl-méthylènoxyméthyl)-acétanilide.

7. Procédé de préparation d'un herbicide, caractérisé par le fait qu'on mélange un support solide ou liquide avec un halogénacétanilide selon la revendication 1.

8. Procédé de lutte contre la croissance indésirable de plantes, caractérisé par le fait qu'on traite les plantes ou le sol avec un halogénacétanilide selon la revendication 1.